Europäisches Patentamt

(19) European Patent Office

Office européen des brevets

(11) Publication number: **0 062 776**
**B1**

(12) **EUROPEAN PATENT SPECIFICATION**

(45) Date of publication of patent specification: **22.05.85**

(21) Application number: **82102211.8**

(22) Date of filing: **18.03.82**

(51) Int. Cl.⁴: **C 07 D 473/12** // A23F5/20, A23F5/22, A61K31/52

(54) **Purification of caffeine.**

(30) Priority: **14.04.81 US 254100**

(43) Date of publication of application:
**20.10.82 Bulletin 82/42**

(45) Publication of the grant of the patent:
**22.05.85 Bulletin 85/21**

(84) Designated Contracting States:
**CH DE FR LI**

(56) References cited:
**CH-A- 114 904**
**GB-A- 854 703**
**US-A-2 508 545**
**US-A-3 321 142**

(73) Proprietor: **SOCIETE DES PRODUITS NESTLE
S.A.**
**Case postale 353**
**CH-1800 Vevey (CH)**

(72) Inventor: **Hirsbrunner, Pierre**
**Route de la Crottaz 30**
**CH-1802 Corseaux (CH)**
Inventor: **Pavillard, Blaise**
**Rue Joseph Chaley 33**
**CH-1700 Fribourg (CH)**

**The file contains technical information
submitted after the application was filed and
not included in this specification**

Courier Press, Leamington Spa, England.

## Description

The present invention relates to a process for the purification of caffeine, more particularly to the production of caffeine in crystalline form which is sufficiently pure for use in pharmaceuticals and foodstuffs.

Caffeine produced industrially by the decaffeination of green coffee is generally rather crude, containing approximately 85% caffeine, and is unsuitable for use in pharmaceuticals or foodstuffs.

Several attempts have been made to purify caffeine. For example, sublimation has been tried but the yield was poor due to chemical decomposition. Purification by recrystallisation from water has also been tried as described in GB—A—854703 but the morphology of the crystals (long needles forming inextricable masses) caused the retention of impure mother-liquor and the chemical nature of the caffeine caused associations with the impurities, preventing the formation of a pure solid phase. Even after four successive recrystallisations a product of the desired purity was not obtained.

In another method, US—A—2508545 describes a process for recovering caffeine from a solution obtained by aqueous extraction of coffee which comprises extracting the aqueous extract with a water-immiscible organic caffeine solvent, redissolving the solute from said organic solvent solution in water, adjusting the pH of the resulting aqueous solution to a value of at least 7.0 by the addition of an alkali base, and crystallising the caffeine therefrom. However, the maximum purity of caffeine stated to be obtained by this process is only 98%.

In yet another method US—A—3321142 describes a process for the preparation of a caffeine granulate in which caffeine is heated above its melting point in the presence of a reducing agent which is chemically inert to caffeine, cooling the caffeine product and grinding to the desired grain size to give a discolouration-free caffeine granulate for use in the pharmceutical industry.

We have found, surprisingly, that if caffeine is recrystallised from an aqueous solution containing alkali and certain reducing agents, a pure caffeine product is obtained which is a much purer product than that obtained by the process of US—A—2508545. In addition if the caffeine is extracted from an aqueous solution containing an alkali by certain water-immiscible organic solvents either before or after recrystallisation a very high yield can be achieved.

Accordingly, the present invention provides a process for the purification of caffeine which comprises carrying out the following steps in any order;

a) recrystallisation of caffeine from an aqueous solution of caffeine containing alkali and a reducing agent and,

b) extraction of the caffeine from an aqueous alkaline solution of caffeine containing alkali with a substantially water immiscible solvent which is liquid under the conditions of the process.

For convenience the expression "aqueous alkaline solution of caffeine containing alkali" in this invention refers to any aqueous solution of caffeine containing alkali.

The pH of the aqueous alkaline solution of the caffeine is preferably from 8 to 10.

The alkali that is contained in the aqueous solution of the caffeine may be any compound, compatible with use in a food environment, that can increase the pH of an aqueous caffeine solution to the desired value. It is preferably inorganic and may be, for example, an alkali metal hydroxide such as sodium hydroxide or potassium hydroxide. The amount used and the concentration of the alkali are conveniently chosen to achieve the desired pH. For example a 30% solution of an alkali metal hydroxide may be used in an amount of from 1 to 20%, preferably from 2.5 to 10% by weight, based on the weight of crude caffeine.

The reducing agent should be of low toxicity, compatible with its use in a food environment. While sulphites may be used, a dithionite such as sodium dithionite, or zinc powder is preferred. The amount of reducing agent may vary widely but usually depends on the particular reducing agent used. For example, a dithionite may be used in an amount of from 2.5 to 15%, preferably from 4 to 10% by weight, based on the weight of crude caffeine, whereas zinc powder may be used in an amount from 0.1 to 10% and preferably from 0.25 to 2.5% by weight based on the weight of crude caffeine.

Desirably, the recrystallisation is carried out in the presence of an inert protecting gas which may be, for example, helium, neon, argon or methane but is preferably nitrogen.

The concentration of caffeine in the aqueous alkaline solution may vary widely, for example, from 5 to 50% but for economic reasons is usually from 7.5 to 25% by weight based on the weight of water.

The temperature of the aqueous alkaline solution of the caffeine is preferably high enough to dissolve all the caffeine present and is conveniently from 50° to 100°C preferably from 60° to 80°C. Recrystallisation takes place by cooling, conveniently to a temperature below 30°C and preferably to a temperature from 15° to 25°C.

The solvent used for the extraction of the caffeine from the aqueous alkaline solution conveniently has a boiling point of from 35° to 100°C and is preferably non-toxic and non-inflammable. The most suitable solvents are halogenated hydrocarbons, preferably chlorinated hydrocarbons, for example, dichloromethane. The extraction is preferably carried out at a temperature at or in the vicinity of the boiling point of the solvent. The amount of solvent used may vary widely and may be from 0.5 to 100 parts and preferably from 1 to 10 parts by weight per part by weight of the aqueous alkaline solution. The extraction of the caffeine with the solvent may suitably take place in a counter-current manner. If the caffeine is extracted after the recrystallisation, it is extracted from the aqueous alkaline solution filtered off from the recrystallised caffeine. If the caffeine is extracted before the recrystallisation, it is preferably extracted from an aqueous alkaline solution of caffeine in the absence of a reducing agent, after which the solvent phase containing the caffeine is separated from the aqueous phase, the solvent evaporated off and the caffeine dissolved in fresh aqueous alkaline solution containing a reducing agent and subsequently recrystallised. The extraction may conveniently be carried out by a continuous process. Preferably the extraction of the caffeine from the aqueous alkaline solution is carried out before the recrystallisation.

By recycling the aqueous alkaline solution containing caffeine and subjecting it to one or more further recrystallisations and extractions it is possible to increase the yield of very pure caffeine. The whole process may be carried out continuously, if desired.

The process of the present invention is preferably carried out at atmospheric pressure. The process can be used to purify caffeine obtained from any source and can easily be adapted to operate in a coffee-manufacturing plant.

The following Examples further illustrate the present invention.

Example 1

100 g of crude industrial caffeine containing 84.8 g caffeine were dissolved in 1 litre of an aqueous solution containing 6 ml of a 30% solution of sodium hydroxide and having a pH of 9 and a temperature of 70°C. To this solution were added 10 g of active charcoal, 5 g of sodium dithionite and 4 g of Celite®. Nitrogen was bubbled through and after mixing, the solution was filtered to remove the active charcoal, Celite® and impurities.

The filtrate was then cooled to 20°C to recrystallise some of the caffeine. The crystals were filtered off and washed with 200 ml of washing water. The mother-liquor, which was an aqueous alkaline solution containing the remainder of the caffeine, together with the washing water, were extracted with 2 litres of dichloromethane at 40°C in a countercurrent manner. The dichloromethane phase containing most of the caffeine was separated from the impure exhausted aqueous alkaline phase and the dichloromethane was evaporated off to yield a further quantity of caffeine, which together with the previously recrystallised caffeine weighed 55 g. This caffeine was added to a mixture of 500 g of fresh water and 1 g of sodium dithionite at 70°C and the pH was adjusted to 9 by further addition of 1.2 ml of 30% sodium hydroxide. Nitrogen was bubbled through and after mixing, the temperature was reduced to 20°C to recrystallise some more of the caffeine. The crystals were filtered off, washed with 200 ml of fresh washing water and dried to give 30 g of pure caffeine. The mother-liquor,

which was an aqueous alkaline solution containing the remainder of the caffeine, together with the washing water, were recirculated and extracted at 40°C with the dichloromethane which had been evaporated off after the first extraction. The dichloromethane phase containing most of the caffeine was separated from the impure aqueous alkaline phase and the dichloromethane was evaporated off and the caffeine recrystallised again as before. This recirculation of the mother-liquor and dichloromethane, and the recrystallisation of the caffeine followed by drying were repeated until the yield of pure caffeine was 95%.

Example 2

100 g of crude industrial caffeine containing 84.8 g caffeine were dissolved in 500 g of an aqueous solution containing 6 ml of a 30% solution of sodium hydroxide and having a pH of 9 and a temperature of 70°C. To this solution were added 10 g of active charcoal, 1 g of powdered zinc and 4 g of Celite®. Nitrogen was bubbled through and after mixing, the solution was filtered to remove the active charcoal, celite and impurities.

The filtrate was then cooled to 20°C to recrystallise some of the caffeine. The crystals were filtered off and washed with 300 ml of washing water. The mother-liquor, which was an aqueous alkaline solution containing the remainder of the caffeine, together with the washing water, were extracted with 1 litre of dichloromethane at 40°C in a countercurrent manner. The dichloromethane phase containing most of the caffeine was separated from the impure exhausted aqueous alkaline phase and the dichloromethane was evaporated off to yield a further quantity of caffeine which together with the previously recrystallised caffeine weighed 44 g. This caffeine was added to a mixture of 850 g of fresh water and 0.4 g of powdered zinc at 70°C and the pH was adjusted to 9 by the further addition of 0.8 g of 30% sodium hydroxide. Nitrogen was bubbled through and after mixing, the temperature was reduced to 20°C to recrystallise some more of the caffeine. The crystals were filtered off, washed with 300 ml of fresh washing water and dried to give 41 g of pure caffeine. The mother-liquor, which was an aqueous alkaline solution containing the remainder of the caffeine, together with the washing water, were recirculated and extracted at 40°C with the dichloromethane which had been evaporated off after the first extraction. The dichloromethane phase containing most of the caffeine was separated from the impure aqueous alkaline phase and the dichloromethane was evaporated off and the caffeine recrystallised again as before. This recirculation of the mother-liquor and dichloromethane, and the recrystallization of the caffeine followed by drying were repeated until the yield of pure caffeine was 95%.

Example 3

100 grams of crude industrial caffeine containing 84.8 g caffeine were dissolved in 1 litre of an aqueous solution containing 6 ml of a 30% solution of sodium hydroxide and mixed at 40°C before filtering to remove the pellicules. The aqueous phase was then extracted with 2 litres of dichloromethane at 40°C in a countercurrent manner up to exhaustion of the caffeine. The dichloromethane phase containing most of the caffeine was separated from the impure aqueous alkaline phase and the dichloromethane was evaporated off leaving 74 g of caffeine. This caffeine was then redissolved in 1100 g of fresh water at 70°C and 0.4 g of 30% solution of sodium hydroxide was added to bring the pH to 9.5. 10 g of activated charcoal, 0.4 g of zinc powder and 4 g of Celite® were then added and nitrogen was bubbled through. After mixing the solution was filtered to remove the zinc, activated charcoal, Celite® and impurities. The filtrate was then cooled to 20°C to recrystallise some of the caffeine. The crystals were filtered off, cleaned with 300 ml of fresh washing water and dried to give 46 g of pure caffeine. The mother-liquor which was an aqueous alkaline solution containing the remainder of the caffeine was recirculated and extracted at 40°C with the dichloromethane which had been evaporated off after the first extraction. The dicloromethane phase containing most of the caffeine which was present in the mother-liquor was separated from the impure aqueous alkaline phase and the dichloromethane was evaporated off and the caffeine recrystallised again as before. This recirculation of the mother-liquor and dichloromethane, and the recrystallisation of the caffeine followed by drying were repeated until the yield of pure caffeine was 95%. The mother-liquor and the washing water can be used to dissolve a fresh charge of crude caffeine and the process repeated.

## Claims

1. A process for the purification of caffeine which comprises carrying out the following steps in any order:
a) recrystallisation of caffeine from an aqueous alkaline solution of caffeine containing alkali and a reducing agent and,
b) extraction of the caffeine from an aqueous alkaline solution of caffeine containing alkali with a substantially water-immiscible solvent which is liquid under the conditions of the process.

2. A process according to claim 1 wherein the pH of the aqueous alkaline solution of caffeine is from 8 to 10.

3. A process according to claim 1 or claim 2 wherein the alkali contained in the aqueous solution of caffeine is an alkali metal hydroxide.

4. A process according to any of the preceding claims wherein the reducing agent is zinc powder.

5. A process according to claim 4 wherein the zinc powder is used in an amount from 0.25 to 2.5% by weight based on the weight of crude caffeine.

6. A process according to any of the preceding claims wherein the recrystallisation is carried out in the presence of nitrogen.

7. A process according to any of the preceding claims wherein the concentration of caffeine in the aqueous alkaline solution is from 7.5 to 25% by weight based on the weight of water.

8. A process according to any of the preceding claims wherein the temperature of the aqueous alkaline solution is from 60° to 80°C.

9. A process according to any of the preceding claims wherein the extraction solvent is a chlorinated hydrocarbon.

10. A process according to any of the preceding claims wherein the extraction solvent is dichloromethane.

11. A process according to any of the preceding claims wherein the extraction of the caffeine from the aqueous alkaline solution is carried out before the recrystallisation.

## Patentansprüche

1. Verfahren zur Reinigung von Coffein, das die Durchführung der folgenden Schritte in beliebiger Reihenfolge umfaßt:
a) Umkristallisation von Coffein aus einer Alkali und ein Reduktionsmittel enthaltenden wäßrigen alkalischen Lösung von Coffein und
b) Extraktion des Coffeins aus einer Alkali enthaltenden wäßrigen alkalischen Lösung von Coffein mit einem im wesentlichen wasserunmischbaren Lösungsmittel, das unter den Bedingungen des Verfahrens flüssig ist.

2. Verfahren nach Anspruch 1, bei dem der pH der wäßrigen alkalischen Lösung des Coffeins von 8 bis 10 beträgt.

3. Verfahren nach Anspruch 1 oder Anspruch 2, bei dem das in der wäßrigen Lösung des Coffeins enthaltene Alkali ein Alkalimetallhydroxid ist.

4. Verfahren nach irgendeinem der vorausgehenden Ansprüche, bei dem das Reduktionsmittel Zinkpulver ist.

5. Verfahren nach Anspruch 4, bei dem das Zinkpulver in einer Menge von 0,25 bis 2,5 Gew.-%, bezogen auf das Gewicht des rohen Coffeins, verwendet wird.

6. Verfahren nach irgendeinem der vorausgehenden Ansprüche, bei dem die Umkristallisation in Gegenwart von Stickstoff durchgeführt wird.

7. Verfahren nach irgendeinem der vorausgehenden Ansprüche, bei dem die Konzentration des Coffeins in der wäßrigen alkalischen Lösung von 7,5 bis 25 Gew.-%, bezogen auf das Gewicht des Wassers, beträgt.

8. Verfahren nach irgendeinem der vorausgehenden Ansprüche, bei dem die Temperatur der wäßrigen alkalischen Lösung von 60 bis 80°C beträgt.

9. Verfahren nach irgendeinem der vorausgehenden Ansprüche, bei dem das Extraktionslösungsmittel ein chlorierter Kohlenwasserstoff ist.

10. Verfahren nach irgendeinem der vorausgehenden Ansprüche, bei dem das Extraktionslösungsmittel Dichlormethan ist.

11. Verfahren nach irgendeinem der vorausgehenden Ansprüche, bei dem die Extraktion des Coffeins aus der wäßrigen alkalischen Lösung vor der Umkristallisation durchgeführt wird.

## Revendications

1. Procédé de purification de caféine, qui consiste à conduire les étapes suivantes dans un ordre quelconque:

a) recristallisation de caféine dans une solution alcaline aqueuse de caféine contenant une base alcaline et un agent réducteur et,

b) extraction de la caféine de la solution alcaline aqueuse de caféine contenant une base alcaline avec un solvant pratiquement non miscible à l'eau qui est liquide dans les conditions du procédé.

2. Procédé suivant la revendication 1, dans lequel le pH de la solution alcaline aqueuse de caféine va de 8 à 10.

3. Procédé suivant la revendication 1 ou la revendication 2, dans lequel la base alcaline présente dans la solution aqueous de caféine est un hydroxyde de métal alcalin.

4. Procédé suivant l'une quelconque des revendications précédentes, dans lequel l'agent réducteur est le zinc en poudre.

5. Procédé suivant la revendication 4, dans lequel le zinc en poudre est utilisé en une quantité de 0,25 à 2,5% en poids sur la base du poids de caféine brute.

6. Procédé suivant l'une quelconque des revendications précédentes, dans lequel la recristallisation est conduite en présence d'azote.

7. Procédé suivant l'une quelconque des revendications précédentes, dans lequel la concentration de caféine dans la solution alcaline aqueuse va de 7,5 à 25% en poids sur la base du poids d'eau.

8. Procédé suivant l'une quelconque des revendications précédentes, dans lequel la température de la solution alcaline aqueuse va de 60 à 80°C.

9. Procédé suivant l'une quelconque des revendications précédentes, dans lequel le solvant d'extraction est un hydrocarbure chloré.

10. Procédé suivant l'une quelconque des revendications précédentes, dans lequel le solvant d'extraction est le dichlorométhane.

11. Procédé suivant l'une quelconque des revendications précédentes, dans lequel l'extraction de la caféine de la solution alcaline aqueuse est effectuée avant la recristallisation.